# EUROPEAN PATENT SPECIFICATION

(11) **EP 4 104 833 B1**
(45) Date of publication and mention of the grant of the patent: **28.02.2024**
(21) Application number: 22178618.9
(22) Date of filing: 13.06.2022
(51) Int. Cl.: A61K 31/375, A61K 31/122, A61K 31/17, A61K 31/175, A61K 31/436, A61K 31/4545, A61K 31/4709, A61K 31/495, A61K 39/395, A61K 45/06, A61P 35/00, A61P 35/02, A61P 35/04

(54) **ASCORBIC ACID AND QUINONE COMPOUND FOR CANCER TREATMENT**
ASCORBINSÄURE UND CHINONVERBINDUNG ZUR KREBSBEHANDLUNG
ACIDE ASCORBIQUE ET QUINONE POUR LE TRAITEMENT DU CANCER

(30) Priority: 14.06.2021 US 202163210405 P
(43) Date of publication of application: 21.12.2022
(73) Proprietor: IC-Medtech Corp., Las Vegas, NV 89158 (US)
(72) Inventor: MILLER, Thomas M., Las Vegas, 89158 (US); ZHELEV, Zhivko, 1408 Sofia (BG)
(74) Representative: Maiwald GmbH

(56) References cited:
- US-A1- 2020 237 710
- Roginsky Vitaly A ET AL: "Ubiquinone-0 (2,3-dimethoxy-5methyl-l,+ benzoquinone) as Effective Catalyzer of Ascorbate and Epinephrine Oxidation and Damager of Neuroblastoma Cells", Biochemical Pharmacology, 1 January 1998 (1998-01-01), pages 85-91, XP055969918, Retrieved from the Internet: URL:https://pubmed.ncbi.nlm.nih.gov/941393 4/ [retrieved on 2022-10-11]
- VERRAX JULIEN ET AL: "Enhancement of quinone redox cycling by ascorbate induces a caspase-3 independent cell death in human leukaemia cells. An in vitro comparative study", FREE RADICAL RESEARCH, vol. 39, no. 6, 7 June 2005 (2005-06-07), pages 649-657, XP055969933, GB ISSN: 1071-5762, DOI: 10.1080/10715760500097906 Retrieved from the Internet: URL:http://dx.doi.org/10.1080/107157605000 97906>
- GROEBER U ET AL: "Vitamin C in der komplementaeren Onkologie", DER ONKOLOGE, SPRINGER, BERLIN, DE, vol. 16, no. 3, 28 February 2010 (2010-02-28), pages 309-313, XP019805486, ISSN: 1433-0415

## Description

### FIELD

Provided herein is a combination of ascorbic acid and 2,3-dimethoxy-5-methyl-1,4-benzoquinone for use in a method of treating or alleviating one or more symptoms of cancer in a subject, comprising administering said compounds intratumorally to the subject in need thereof.

### BACKGROUND

Cancer is a major worldwide public health problem. It was estimated that there will be 1,898,160 new cancer cases diagnosed and 608,570 cancer deaths in the US alone in 2021. *Cancer Facts & Figures* 2021. Despite many advances in cancer treatment, cancer such as brain cancer, liver cancer, lung cancer, myeloid malignancies, ovarian cancer, pancreatic cancer, and stomach cancer, especially at an advanced stage, has limited treatment options. Allemani et al., Lancet 2018, 391, 1023-75; Lai et al., Cancer Treat. Rev. 2019, 81, 101926; Aldape et al., Nat. Rev. Clin. Oncol. 2019, 16, 509-20; Gedeon et al., Expert Rev. Clin. Pharmacol. 2020, 13, 1147-58. For example, glioblastoma multiforme (GBM), the deadliest form of brain cancer, has a median survival rate of less than 2 years after diagnosis and an average 5-year survival rate of less than 5%. Batash et al., Curr. Med. Chem. 2017, 24, 3002-9; Jackson et al., Nat. Immunol. 2019, 20, 1100-9. However, no new, more effective therapies for GBM have been developed in the past 30 years. Batash etal., Curr. Med. Chem. 2017, 24, 3002-9; Aldape et al., Nat. Rev. Clin. Oncol. 2019, 16, 509-20; Gedeon et al., Expert Rev. Clin. Pharmacol. 2020, 13, 1147-58. US 2020/0237710 relates to a method of treating cancer in a subject comprising the administration of vitamin C and vitamin K1.

Therefore, there is a need for effective treatment for cancer.

### SUMMARY OF THE DISCLOSURE

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

Provided herein is a combination of (i) ascorbic acid, or a diastereomer, a mixture of two or more diastereomers, a tautomer, a mixture of two or more tautomers; or a pharmaceutically acceptable salt, solvate, or hydrate thereof; and (ii) a quinone compound, or a tautomer, a mixture of two or more tautomers, or an isotopic variant thereof; or a pharmaceutically acceptable salt, solvate or hydrate thereof; wherein the quinone compound is 2,3-dimethoxy-5-methyl-1,4-benzoquinone; for use in a method of treating or alleviating one or more symptoms of cancer in a subject, comprising administering intratumorally to the subject in need thereof a therapeutically effective amount of (i) and (ii).

### BRIEF DESCRIPTION OF THE DRAWINGS

FIG. 1 shows the antitumor effect of vitamin C and compound **A6** against glioblastoma in a xenograft mouse model, where vitamin C and compound **A6** were administered intracranially once on Day 7 (*i.e.,* 6 days after the implantation of glioblastoma cells).
FIG. 2 shows a survival curve of glioblastoma mice in a xenograft mouse model, where vitamin C and compound **A6** were administered intracranially once on Day 7 (*i.e.,* 6 days after the implantation of glioblastoma cells).
FIG. 3 shows the antitumor effect of vitamin C and compound **A6** against glioblastoma in a xenograft mouse model, where vitamin C and compound **A6** were administered intratumorally once on Day 4 (*i.e.,* 3 days after the implantation of glioblastoma cells); or twice, once each on Days 4 and 10 (*i.e.,* 3 and 9 days, respectively, after the implantation of glioblastoma cells).

### DETAILED DESCRIPTION

To facilitate understanding of the disclosure set forth herein, a number of terms are defined below.

Generally, the nomenclature used herein and the laboratory procedures in organic chemistry, medicinal chemistry, biochemistry, biology, and pharmacology described herein are those well-known and commonly employed in the art. Unless defined otherwise, all technical and scientific terms used herein generally have the same meaning as commonly understood by one of ordinary skill in the art to which this disclosure belongs.

The term "subject" refers to an animal, including, but not limited to, a primate (*e.g.*, human), livestock, a domestic pet, cow, pig, sheep, goat, horse, dog, cat, rabbit, rat, or mouse. The terms "subject" and "patient" are used interchangeably herein in reference, for example, to a mammalian subject, such as a human subject, in one embodiment, a human.

The terms "treat," "treating," and "treatment" are meant to include alleviating or abrogating a disorder, disease, or condition, or one or more of the symptoms associated with the disorder, disease, or condition; or alleviating or eradicating the cause(s) of the disorder, disease, or condition itself.

The terms "prevent," "preventing," and "prevention" are meant to include a method of delaying and/or precluding the onset of a disorder, disease, or condition, and/or its attendant symptoms; barring a subject from acquiring a disorder, disease, or condition; or reducing a subject's risk of acquiring a disorder, disease, or condition.

The terms "alleviate" and "alleviating" refer to easing or reducing one or more symptoms (*e.g.*, pain) of a disorder, disease, or condition. The terms can also refer to reducing adverse effects associated with an active ingredient. Sometimes, the beneficial effects that a subject derives from a prophylactic or therapeutic agent do not result in a cure of the disorder, disease, or condition.

The term "contacting" or "contact" is meant to refer to bringing together of a therapeutic agent and a biological molecule (*e.g.,* a protein, enzyme, RNA, or DNA), cell, or tissue such that a physiological and/or chemical effect takes place as a result of such contact. Contacting can take place *in vitro, ex vivo,* or *in vivo.* In one embodiment, a therapeutic agent is contacted with a biological molecule *in vitro* to determine the effect of the therapeutic agent on the biological molecule. In another embodiment, a therapeutic agent is contacted with a cell in cell culture (*in vitro*) to determine the effect of the therapeutic agent on the cell. In yet another embodiment, the contacting of a therapeutic agent with a biological molecule, cell, or tissue includes the administration of a therapeutic agent to a subject having the biological molecule, cell, or tissue to be contacted.

The terms "therapeutically effective amount" and "effective amount" are meant to include the amount of a compound or a combination of compounds that, when administered, is sufficient to prevent development of, or alleviate to some extent, one or more of the symptoms of the disorder, disease, or condition being treated. The term "therapeutically effective amount" or "effective amount" also refers to the amount of a compound or a combination of compounds that is sufficient to elicit the biological or medical response of a biological molecule (*e.g.,* a protein, enzyme, RNA, or DNA), cell, tissue, system, animal, or human, which is being sought by a researcher, veterinarian, medical doctor, or clinician.

The term "IC₅₀" or "EC₅₀" refers to an amount, concentration, or dosage of a compound that is required for 50% inhibition of a maximal response in an assay that measures such a response.

The term "pharmaceutically acceptable carrier," "pharmaceutically acceptable excipient," "physiologically acceptable carrier," or "physiologically acceptable excipient" refers to a pharmaceutically acceptable material, composition, or vehicle, such as a liquid or solid filler, diluent, solvent, or encapsulating material. In one embodiment, each component is "pharmaceutically acceptable" in the sense of being compatible with the other ingredients of a pharmaceutical formulation, and suitable for use in contact with the tissue or organ of a subject (*e.g.,* a human) without excessive toxicity, irritation, allergic response, immunogenicity, or other problems or complications, and commensurate with a reasonable benefit/risk ratio. *See, e.g.,* Remington: The Science and Practice of Pharmacy, 23rd ed.; Adejare Ed.; Academic Press, 2020; Handbook of Pharmaceutical Excipients, 9th ed.; Sheskey et al., Eds.; Pharmaceutical Press, 2020; Handbook of Pharmaceutical Additives, 3rd ed.; Ash and Ash Eds.; Synapse Information Resources, 2007; Pharmaceutical Preformulation and Formulation, 1st ed.; Gibson Ed.; CRC Press, 2015.

The term "about" or "approximately" means an acceptable error for a particular value as determined by one of ordinary skill in the art, which depends in part on how the value is measured or determined. In certain embodiments, the term "about" or "approximately" means within 1, 2, or 3 standard deviations. In certain embodiments, the term "about" or "approximately" means within 25%, 20%, 15%, 10%, 9%, 8%, 7%, 6%, 5%, 4%, 3%, 2%, 1%, 0.5%, or 0.05% of a given value or range.

The terms "active ingredient" and "active substance" refer to a compound, which is administered, alone or in combination with one or more pharmaceutically acceptable excipients, to a subject for treating, preventing, or ameliorating one or more symptoms of a disorder, disease, or condition. As used herein, "active ingredient" and "active substance" may be an optically active isomer of a compound described herein.

In certain embodiments, "optically active" and "enantiomerically active" refer to a collection of molecules, which has an enantiomeric excess of no less than about 80%, no less than about 90%, no less than about 91%, no less than about 92%, no less than about 93%, no less than about 94%, no less than about 95%, no less than about 96%, no less than about 97%, no less than about 98%, no less than about 99%, no less than about 99.5%, or no less than about 99.8%. In certain embodiments, an optically active compound comprises about 95% or more of one enantiomer and about 5% or less of the other enantiomer based on the total weight of the enantiomeric mixture in question. In certain embodiments, an optically active compound comprises about 98% or more of one enantiomer and about 2% or less of the other enantiomer based on the total weight of the enantiomeric mixture in question. In certain embodiments, an optically active compound comprises about 99% or more of one enantiomer and about 1% or less of the other enantiomer based on the total weight of the enantiomeric mixture in question.

In describing an optically active compound, the prefixes *R* and *S* are used to denote the absolute configuration of the compound about its chiral center(s). The (+) and (-) are used to denote the optical rotation of the compound, that is, the direction in which a plane of polarized light is rotated by the optically active compound. The (-) prefix indicates that the compound is levorotatory, that is, the compound rotates the plane of polarized light to the left or counterclockwise. The (+) prefix indicates that the compound is dextrorotatory, that is, the compound rotates the plane of polarized light to the right or clockwise. However, the sign of optical rotation, (+) and (-), is not related to the absolute configuration of the compound, *R* and *S.*

The term "isotopically enriched" refers to a compound that contains an unnatural proportion of an isotope at one or more of the atoms that constitute such a compound. In certain embodiments, an isotopically enriched compound contains unnatural proportions of one or more isotopes, including, but not limited to, hydrogen (³H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain embodiments, an isotopically enriched compound is in a stable form, that is, non-radioactive. In certain embodiments, an isotopically enriched compound contains unnatural proportions of one or more isotopes, including, but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), oxygen-18 (¹⁸O), fluorine-17 (¹⁷F), phosphorus-31 (³¹P), sulfur-32 (³²S), sulfur-33 (³³S), sulfur-34 (³⁴S), sulfur-36 (³⁶S), chlorine-35 (³⁵Cl), chlorine-37 (¹⁷Cl), bromine-79 (⁷⁹Br), bromine-81 (⁸¹Br), and iodine-127 (¹²⁷I). In certain embodiments, an isotopically enriched compound is in an unstable form, that is, radioactive. In certain embodiments, an isotopically enriched compound contains unnatural proportions of one or more isotopes, including, but not limited to, tritium (³H), carbon-11 (¹¹C), carbon-14 (¹⁴C), nitrogen-13 (¹³N), oxygen-14 (¹⁴O), oxygen-15 (¹⁵O), fluorine-18 (¹⁸F), phosphorus-32 (³²P), phosphorus-33 (³³P), sulfur-35 (³⁵S), chlorine-36 (³⁶Cl), iodine-123 (¹²³I), iodine-125 (¹²⁵I), iodine-129 (¹²⁹I), and iodine-131 (¹³¹I). It will be understood that, in a compound as provided herein, any hydrogen can be ²H, as example, or any carbon can be ¹³C, as example, or any nitrogen can be ¹⁵N, as example, or any oxygen can be ¹⁸O, as example, where feasible according to the judgment of one of ordinary skill in the art.

The term "isotopic enrichment" refers to the percentage of incorporation of a less prevalent isotope (*e.g.,* D for deuterium or hydrogen-2) of an element at a given position in a molecule in the place of a more prevalent isotope (*e.g.,* ¹H for protium or hydrogen-1) of the element. As used herein, when an atom at a particular position in a molecule is designated as a particular less prevalent isotope, it is understood that the abundance of that isotope at that position is substantially greater than its natural abundance.

The term "isotopic enrichment factor" refers the ratio between the isotopic abundance in an isotopically enriched compound and the natural abundance of a specific isotope.

The term "hydrogen" or the symbol "H" refers to the composition of naturally occurring hydrogen isotopes, which include protium (¹H), deuterium (²H or D), and tritium (³H), in their natural abundances. Protium is the most common hydrogen isotope having a natural abundance of more than 99.98%. Deuterium is a less prevalent hydrogen isotope having a natural abundance of about 0.0156%.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156% on average, deuterium enrichment at any position in a compound synthesized using non-enriched starting materials is about 0.0156% on average. As used herein, when a particular position in an isotopically enriched compound is designated as having deuterium, it is understood that the abundance of deuterium at that position in the compound is substantially greater than its natural abundance (0.0156%).

The term "carbon" or the symbol "C" refers to the composition of naturally occurring carbon isotopes, which include carbon-12 (¹²C) and carbon-13 (¹³C) in their natural abundances. Carbon-12 is the most common carbon isotope having a natural abundance of more than 98.89%. Carbon-13 is a less prevalent carbon isotope having a natural abundance of about 1.11%.

The term "carbon-13 enrichment" or "¹³C enrichment" refers to the percentage of incorporation of carbon-13 at a given position in a molecule in the place of carbon. For example, carbon-13 enrichment of 10% at a given position means that 10% of molecules in a given sample contain carbon-13 at the specified position. Because the naturally occurring distribution of carbon-13 is about 1.11 % on average, carbon-13 enrichment at any position in a compound synthesized using non-enriched starting materials is about 1.11% on average. As used herein, when a particular position in an isotopically enriched compound is designated as having carbon-13, it is understood that the abundance of carbon-13 at that position in the compound is substantially greater than its natural abundance (1.11%).

The terms "substantially pure" and "substantially homogeneous" mean, when referred to a substance, sufficiently homogeneous to appear free of readily detectable impurities as determined by a standard analytical method used by one of ordinary skill in the art, including, but not limited to, thin layer chromatography (TLC), gel electrophoresis, high performance liquid chromatography (HPLC), gas chromatography (GC), nuclear magnetic resonance (NMR), and mass spectrometry (MS); or sufficiently pure such that further purification would not detectably alter the physical, chemical, biological, and/or pharmacological properties, such as enzymatic and biological activities, of the substance. In certain embodiments, "substantially pure" or "substantially homogeneous" refers to a collection of molecules, wherein at least about 95%, at least about 96%, at least about 97%, at least about 98%, at least about 99%, or at least about 99.5% by weight of the molecules are a single compound, including a single enantiomer, a racemic mixture, or a mixture of enantiomers, as determined by standard analytical methods. As used herein, when an atom at a particular position in an isotopically enriched molecule is designated as a particular less prevalent isotope, a molecule that contains other than the designated isotope at the specified position is an impurity with respect to the isotopically enriched compound. Thus, for a deuterated compound that has an atom at a particular position designated as deuterium, a compound that contains a protium at the same position is an impurity.

The term "solvate" refers to a complex or aggregate formed by one or more molecules of a solute, *e.g.,* a compound provided herein, and one or more molecules of a solvent, which are present in stoichiometric or non-stoichiometric amount. Suitable solvents include, but are not limited to, water, methanol, ethanol, n-propanol, isopropanol, and acetic acid. In certain embodiments, the solvent is pharmaceutically acceptable. In one embodiment, the complex or aggregate is in a crystalline form. In another embodiment, the complex or aggregate is in a noncrystalline form. Where the solvent is water, the solvate is a hydrate. Examples of hydrates include, but are not limited to, a hemihydrate, monohydrate, dihydrate, trihydrate, tetrahydrate, and pentahydrate.

The phrase "an enantiomer, a mixture of enantiomers, a mixture of two or more diastereomers, a tautomer, a mixture of two or more tautomers, or an isotopic variant thereof; or a pharmaceutically acceptable salt, solvate, hydrate, or prodrug thereof' has the same meaning as the phrase "(i) an enantiomer, a mixture of enantiomers, a mixture of two or more diastereomers, a tautomer, a mixture of two or more tautomers, or an isotopic variant of the compound referenced therein; (ii) a pharmaceutically acceptable salt, solvate, hydrate, or prodrug of the compound referenced therein; or (iii) a pharmaceutically acceptable salt, solvate, hydrate, or prodrug of an enantiomer, a mixture of enantiomers, a mixture of two or more diastereomers, a tautomer, a mixture of two or more tautomers, or an isotopic variant of the compound referenced therein."

### Ascorbic Acid Compound

In one embodiment, the ascorbic acid compound is L-ascorbic acid or a pharmaceutically acceptable salt thereof; or a pharmaceutically acceptable solvate or hydrate thereof. In another embodiment, the ascorbic acid compound is vitamin C, L-xyloascorbic acid, 3-oxo-L-gulofuranolactone (enol form), L-3-ketothreohexuronic acid lactone, antiscorbutic vitamin, cevitamic acid, adenex, allercorb, ascorin, ascorteal, ascorvit, cantan, cantaxin, catavin C, cebicure, cebion, cecon, cegiolan, celaskon, celin, cenetone, cereon, cergona, cescorbat, cetamid, cetabe, cetemican, cevalin, cevatine, cevex, cevimin, ce-vi-sol, cevitan, cevitex, cewin, ciamin, cipca, concemin, C-vin, daviamon C, duoscorb, hybrin, laroscorbine, lemascorb, planavit C, proscorbin, redoxon, ribena, scorbacid, scorbu-C, testascorbic, vicelat, vitacee, vitacimin, vitacin, vitascorbol, or xitix. In yet another embodiment, the ascorbic acid compound is L-ascorbic acid. In still another embodiment, the ascorbic acid compound is a pharmaceutically acceptable salt of L-ascorbic acid, or a pharmaceutically acceptable solvate or hydrate thereof.

Suitable bases for forming a pharmaceutically acceptable salt of L-ascorbic acid include, but are not limited to, inorganic bases, such as magnesium hydroxide, calcium hydroxide, potassium hydroxide, zinc hydroxide, and sodium hydroxide; and organic bases, such as primary, secondary, tertiary, and quaternary, aliphatic and aromatic amines, including, but not limited to, L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, *N*-methyl-glucamine, hydrabamine, 1*H-*imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, triethanolamine, trimethylamine, triethylamine, *N*-methyl-D-glucamine, 2-amino-2-(hydroxymethyl)-1,3-propanediol, and tromethamine.

In one embodiment, the ascorbic acid compound is an alkali or alkaline earth metal salt of L-ascorbic acid, or a pharmaceutically acceptable solvate or hydrate thereof. In another embodiment, the ascorbic acid compound is sodium, potassium, calcium, or magnesium L-ascorbate, or a pharmaceutically acceptable solvate or hydrate thereof. In yet another embodiment, the ascorbic acid compound is sodium L-ascorbate, or a pharmaceutically acceptable solvate or hydrate thereof. In yet another embodiment, the ascorbic acid compound is sodium L-ascorbate. In yet another embodiment, the ascorbic acid compound is vitamin C sodium, ascorbin, sodascorbate, natrascorb, cenolate, ascorbicin, or cebitate. In yet another embodiment, the ascorbic acid compound is potassium L-ascorbate, or a pharmaceutically acceptable solvate or hydrate thereof. In yet another embodiment, the ascorbic acid compound is calcium L-ascorbate, or a pharmaceutically acceptable solvate or hydrate thereof. In yet another embodiment, the ascorbic acid compound is calcium L-ascorbate. In yet another embodiment, the ascorbic acid compound is magnesium L-ascorbate, or a pharmaceutically acceptable solvate or hydrate thereof. In still another embodiment, the ascorbic acid compound is magnesium L-ascorbate.

In certain embodiments, the ascorbic acid compound is D-ascorbic acid or a pharmaceutically acceptable salt, or a pharmaceutically acceptable solvate or hydrate thereof.

In one embodiment, the ascorbic acid compound is D-ascorbic acid. In another embodiment, the ascorbic acid compound is a pharmaceutically acceptable salt of D-ascorbic acid, or a pharmaceutically acceptable solvate or hydrate thereof.

Suitable bases for forming a pharmaceutically acceptable salt of D-ascorbic acid include, but are not limited to, inorganic bases, such as magnesium hydroxide, calcium hydroxide, potassium hydroxide, zinc hydroxide, and sodium hydroxide; and organic bases, such as primary, secondary, tertiary, and quaternary, aliphatic and aromatic amines, including, but not limited to, L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, *N*-methyl-glucamine, hydrabamine, 1*H-*imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, triethanolamine, trimethylamine, triethylamine, *N*-methyl-D-glucamine, 2-amino-2-(hydroxymethyl)-1,3-propanediol, and tromethamine.

In one embodiment, the ascorbic acid compound is an alkali or alkaline earth metal salt of D-ascorbic acid, or a pharmaceutically acceptable solvate or hydrate thereof. In another embodiment, the ascorbic acid compound is sodium, potassium, calcium, or magnesium D-ascorbate, or a pharmaceutically acceptable solvate or hydrate thereof. In yet another embodiment, the ascorbic acid compound is sodium D-ascorbate, or a pharmaceutically acceptable solvate or hydrate thereof. In yet another embodiment, the ascorbic acid compound is sodium D-ascorbate. In yet another embodiment, the ascorbic acid compound is potassium D-ascorbate, or a pharmaceutically acceptable solvate or hydrate thereof. In yet another embodiment, the ascorbic acid compound is calcium D-ascorbate, or a pharmaceutically acceptable solvate or hydrate thereof. In yet another embodiment, the ascorbic acid compound is calcium D-ascorbate. In yet another embodiment, the ascorbic acid compound is magnesium D-ascorbate, or a pharmaceutically acceptable solvate or hydrate thereof. In still another embodiment, the ascorbic acid compound is magnesium D-ascorbate.

### Quinone Compounds

The quinone compound described herein is 2,3-dimethoxy-5-methyl-1,4-benzoquinone **A6**, or a tautomer, a mixture of two or more tautomers, or an isotopic variant thereof; or a pharmaceutically acceptable salt, solvate or hydrate thereof.

### Pharmaceutical Compositions

In one embodiment, provided herein is a pharmaceutical composition comprising (i) ascorbic acid, or a diastereomer, a mixture of two or more diastereomers, a tautomer, a mixture of two or more tautomers; or a pharmaceutically acceptable salt, solvate, or hydrate thereof; and (ii) a quinone compound, or a tautomer, a mixture of two or more tautomers, or an isotopic variant thereof; or a pharmaceutically acceptable salt, solvate or hydrate thereof; wherein the quinone compound is 2,3-dimethoxy-5-methyl-1,4-benzoquinone.

In certain embodiments, the pharmaceutical composition further comprises a pharmaceutically acceptable excipient.

In one embodiment, the weight ratio of the ascorbic acid to the quinone compound in a pharmaceutical composition provided herein is ranging from about 4 to about 500, from about 10 to about 500, from about 50 to about 500, from about 25 to about 250, from about 50 to about 200, from about 50 to about 150, or from about 80 to about 120. In another embodiment, the weight ratio of the ascorbic acid to the quinone compound in a pharmaceutical composition provided herein is ranging from about 4 to about 500. In yet another embodiment, the weight ratio of the ascorbic acid to the quinone compound in a pharmaceutical composition provided herein is ranging from about 10 to about 500. In yet another embodiment, the weight ratio of the ascorbic acid to the quinone compound in a pharmaceutical composition provided herein is ranging from about 50 to about 500. In yet another embodiment, the weight ratio of the ascorbic acid to the quinone compound in a pharmaceutical composition provided herein is ranging from about 25 to about 250. In yet another embodiment, the weight ratio of the ascorbic acid to the quinone compound in a pharmaceutical composition provided herein is ranging from about 50 to about 200. In yet another embodiment, the weight ratio of the ascorbic acid to the quinone compound in a pharmaceutical composition provided herein is ranging from about 50 to about 150. In still another embodiment, the weight ratio of the ascorbic acid to the quinone compound in a pharmaceutical composition provided herein is ranging from about 80 to about 120.

In one embodiment, the weight ratio of the ascorbic acid to the quinone compound in a pharmaceutical composition provided herein is about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, or about 250. In another embodiment, the weight ratio of the ascorbic acid to the quinone compound in a pharmaceutical composition provided herein is about 50. In yet another embodiment, the weight ratio of the ascorbic acid to the quinone compound in a pharmaceutical composition provided herein is about 100. In still another embodiment, the weight ratio of the ascorbic acid to the quinone compound in a pharmaceutical composition provided herein is about 200.

In one embodiment, the molar ratio of the ascorbic acid to the quinone compound in a pharmaceutical composition provided herein is ranging from about 10 to about 500, from about 25 to about 250, from about 50 to about 200, from about 50 to about 150, or from about 80 to about 120. In another embodiment, the molar ratio of the ascorbic acid to the quinone compound in a pharmaceutical composition provided herein is ranging from about 10 to about 500. In yet another embodiment, the molar ratio of the ascorbic acid to the quinone compound in a pharmaceutical composition provided herein is ranging from about 25 to about 250. In yet another embodiment, the molar ratio of the ascorbic acid to the quinone compound in a pharmaceutical composition provided herein is ranging from about 50 to about 200. In yet another embodiment, the molar ratio of the ascorbic acid to the quinone compound in a pharmaceutical composition provided herein is ranging from about 50 to about 150. In still another embodiment, the molar ratio of the ascorbic acid to the quinone compound in a pharmaceutical composition provided herein is ranging from about 80 to about 120.

In one embodiment, the molar ratio of the ascorbic acid to the quinone compound in a pharmaceutical composition provided herein is about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, or about 250. In another embodiment, the molar ratio of the ascorbic acid to the quinone compound in a pharmaceutical composition provided herein is about 50. In yet another embodiment, the molar ratio of the ascorbic acid to the quinone compound in a pharmaceutical composition provided herein is about 100. In still another embodiment, the molar ratio of the ascorbic acid to the quinone compound in a pharmaceutical composition provided herein is about 200.

In certain embodiments, a pharmaceutical composition provided herein is formulated as a modified release dosage form, including, but not limited to, a delayed-, extended-, prolonged-, sustained-, pulsatile-, controlled-, accelerated-, fast-, targeted-, or programmed-release form. These dosage forms can be prepared according to conventional methods and techniques known to those skilled in the art (S*ee*, *e.g., Remington: The Science and Practice of Pharmacy, supra;* Modified-Release Drug Delivery Technology, Rathbone et al., Eds., Drugs and the Pharmaceutical Sciences, CRC Press LLC: 2008; Vol. 183).

The pharmaceutical composition provided herein is formulated for intratumoral administration. In certain embodiments, a pharmaceutical composition provided herein is formulated for intratumoral infusion. In certain embodiments, a pharmaceutical composition provided herein is formulated for intratumoral administration *via* direct injection. In certain embodiments, a pharmaceutical composition provided herein is formulated for intratumoral administration *via* implantation. In certain embodiments, a pharmaceutical composition provided herein is formulated for intratumoral administration *via* intracavitary delivery. In certain embodiments, a pharmaceutical composition provided herein is formulated for intratumoral administration *via* intracerebral delivery. In certain embodiments, a pharmaceutical composition provided herein is formulated for intratumoral administration *via* intracranial delivery. In certain embodiments, a pharmaceutical composition provided herein is formulated for intratumoral administration *via* intrathecal delivery. In certain embodiments, a pharmaceutical composition provided herein is formulated for intratumoral administration *via* intraventricular delivery. In certain embodiments, a pharmaceutical composition provided herein is formulated for intratumoral administration *via* convection-enhanced delivery (CED).

In one embodiment, the ascorbic acid in a pharmaceutical composition provided herein is L-ascorbic acid or a pharmaceutically acceptable salt thereof, or a pharmaceutically acceptable solvate or hydrate thereof. In another embodiment, the ascorbic acid in a pharmaceutical composition provided herein is an alkali or alkaline earth metal salt of L-ascorbic acid, or a pharmaceutically acceptable solvate or hydrate thereof; or a mixture thereof. In yet another embodiment, the ascorbic acid in a pharmaceutical composition provided herein is sodium, potassium, calcium, or magnesium salt of L-ascorbic acid, or a pharmaceutically acceptable solvate or hydrate thereof; or a mixture thereof. In yet another embodiment, the ascorbic acid in a pharmaceutical composition provided herein is sodium L-ascorbate. In yet another embodiment, the ascorbic acid in a pharmaceutical composition provided herein is calcium L-ascorbate. In yet another embodiment, the ascorbic acid in a pharmaceutical composition provided herein is magnesium L-ascorbate. In still another embodiment, the ascorbic acid in a pharmaceutical composition provided herein is a mixture of two or three of sodium L-ascorbate, calcium L-ascorbate, and magnesium L-ascorbate.

The quinone compound in a pharmaceutical composition provided herein is 2,3-dimethoxy-5-methyl-1,4-benzoquinone **A6**, or a tautomer, a mixture of two or more tautomers, or an isotopic variant thereof; or a pharmaceutically acceptable salt, solvate or hydrate thereof.

In certain embodiments, a pharmaceutical composition provided herein is provided in a unit-dosage or multiple-dosage form. A unit-dosage form, as used herein, refers to a physically discrete unit suitable for administration to a subject, *e.g.*, a human and animal subject, and packaged individually as is known in the art. Each unit-dose contains a predetermined quantity of one or more active ingredient(s) sufficient to produce the desired therapeutic effect, optionally in association with one or more pharmaceutical vehicle(s), carrier(s), diluent(s), or excipient(s). Examples of a unit-dosage form include an ampoule, syringe, and individually packaged tablet and capsule. A unit-dosage form may be administered in fractions or multiples thereof. A multiple-dosage form is a plurality of identical unit-dosage forms packaged in a single container to be administered in segregated unit-dosage form.

A pharmaceutical composition provided herein may be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations. Methods of Use

Provided herein is a combination of (i) ascorbic acid, or a diastereomer, a mixture of two or more diastereomers, a tautomer, a mixture of two or more tautomers; or a pharmaceutically acceptable salt, solvate, or hydrate thereof; and (ii) a quinone compound, or a tautomer, a mixture of two or more tautomers, or an isotopic variant thereof; or a pharmaceutically acceptable salt, solvate or hydrate thereof; wherein the quinone compound is 2,3-dimethoxy-5-methyl-1,4-benzoquinone; for use in a method of treating or alleviating one or more symptoms of cancer in a subject, comprising administering intratumorally to the subject in need thereof a therapeutically effective amount of (i) and (ii).

In certain embodiments, the cancer is bladder cancer, brain cancer, breast cancer, colon cancer, endometrial cancer, kidney cancer, leukemia, liver cancer, lung cancer, lymphoma, melanoma, a myeloid malignancy, non-Hodgkin lymphoma, ovarian cancer, pancreatic cancer, prostate cancer, rectal cancer, stomach cancer, or thyroid cancer. In certain embodiments, the cancer is brain cancer, breast cancer, colon cancer, liver cancer, leukemia, lung cancer, lymphoma, a myeloid malignancy, ovarian cancer, pancreatic cancer, or stomach cancer. In certain embodiments, the cancer is brain cancer, liver cancer, lung cancer, a myeloid malignancy, ovarian cancer, pancreatic cancer, or stomach cancer. In certain embodiments, the cancer is brain cancer, breast cancer, colon cancer, leukemia, or lymphoma.

In certain embodiments, the cancer is refractory and/or relapsed. In certain embodiments, the cancer is refractory. In certain embodiments, the cancer is relapsed. In certain embodiments, the cancer is recurrent. In certain embodiments, the cancer is metastatic. In certain embodiments, the cancer is drug-resistant. In certain embodiments, the cancer is multidrug-resistant.

In certain embodiments, the cancer is a solid cancer. In certain embodiments, the solid cancer is refractory and/or relapsed. In certain embodiments, the solid cancer is refractory. In certain embodiments, the solid cancer is relapsed. In certain embodiments, the solid cancer is recurrent. In certain embodiments, the solid cancer is metastatic. In certain embodiments, the solid cancer is drug-resistant. In certain embodiments, the solid cancer is multidrug-resistant. In certain embodiments, the solid cancer is resectable. In certain embodiments, the solid cancer is unresectable.

In certain embodiments, the solid cancer is a brain cancer. In certain embodiments, the brain cancer is refractory and/or relapsed. In certain embodiments, the brain cancer is refractory. In certain embodiments, the brain cancer is relapsed. In certain embodiments, the brain cancer is recurrent. In certain embodiments, the brain cancer is metastatic. In certain embodiments, the brain cancer is drug-resistant. In certain embodiments, the brain cancer is multidrug-resistant. In certain embodiments, the brain cancer is resectable. In certain embodiments, the brain cancer is unresectable.

In certain embodiments, the brain cancer is a primary brain cancer. In certain embodiments, the brain cancer is a secondary or metastatic brain cancer.

In certain embodiments, the brain cancer is a glioma. In certain embodiments, the glioma is refractory and/or relapsed. In certain embodiments, the glioma is refractory. In certain embodiments, the glioma is relapsed. In certain embodiments, the glioma is recurrent. In certain embodiments, the glioma is metastatic. In certain embodiments, the glioma is drug-resistant. In certain embodiments, the glioma is multidrug-resistant. In certain embodiments, the glioma is resectable. In certain embodiments, the glioma is unresectable.

In certain embodiments, the brain cancer is anaplastic astrocytoma, anaplastic ependymoma, anaplastic oligodendroglioma, craniopharyngioma, diffuse astrocytoma, ependymoblastoma, gangliocytoma, ganglioglioma, glioblastoma, medulloblastoma, pineoblastoma, pineocytoma, pilocytic astrocytoma, or pure oligodendroglioma. In certain embodiments, the brain cancer is astrocytoma, glioblastoma or glioblastoma multiforme (GBM), meningioma, or medulloblastoma.

In certain embodiments, the brain cancer is astrocytoma. In certain embodiments, the astrocytoma is refractory and/or relapsed. In certain embodiments, the astrocytoma is refractory. In certain embodiments, the astrocytoma is relapsed. In certain embodiments, the astrocytoma is recurrent. In certain embodiments, the astrocytoma is metastatic. In certain embodiments, the astrocytoma is drug-resistant. In certain embodiments, the astrocytoma is multidrug-resistant. In certain embodiments, the astrocytoma is resectable. In certain embodiments, the astrocytoma is unresectable.

In certain embodiments, the brain cancer is glioblastoma. In certain embodiments, the glioblastoma is refractory and/or relapsed. In certain embodiments, the glioblastoma is refractory. In certain embodiments, the glioblastoma is relapsed. In certain embodiments, the glioblastoma is recurrent. In certain embodiments, the glioblastoma is metastatic. In certain embodiments, the glioblastoma is drug-resistant. In certain embodiments, the glioblastoma is multidrug-resistant. In certain embodiments, the glioblastoma is resectable. In certain embodiments, the glioblastoma is unresectable.

In certain embodiments, the brain cancer is meningioma. In certain embodiments, the meningioma is refractory and/or relapsed. In certain embodiments, the meningioma is refractory. In certain embodiments, the meningioma is relapsed. In certain embodiments, the meningioma is recurrent. In certain embodiments, the meningioma is metastatic. In certain embodiments, the meningioma is drug-resistant. In certain embodiments, the meningioma is multidrug-resistant. In certain embodiments, the meningioma is resectable. In certain embodiments, the meningioma is unresectable.

In certain embodiments, the brain cancer is medulloblastoma. In certain embodiments, the medulloblastoma is refractory and/or relapsed. In certain embodiments, the medulloblastoma is refractory. In certain embodiments, the medulloblastoma is relapsed. In certain embodiments, the medulloblastoma is recurrent. In certain embodiments, the medulloblastoma is metastatic. In certain embodiments, the medulloblastoma is drug-resistant. In certain embodiments, the medulloblastoma is multidrug-resistant. In certain embodiments, the medulloblastoma is resectable. In certain embodiments, the medulloblastoma is unresectable.

In certain embodiments, the ascorbic acid and quinone compound as used in a method provided herein are delivered as a single dose, such as, *e.g.,* as a single bolus injection. In certain embodiments, the ascorbic acid and quinone compound as used in a method provided herein are administered over time, such as, *e.g*., continuous infusion over time or divided bolus doses over time.

In certain embodiments, the weight ratio of the ascorbic acid to the quinone compound as used in a method provided herein is ranging from about 1 to about 1,000, from about 10 to about 500, from about 50 to about 500, from about 100 to about 500, from about 200 to about 500, or from about 200 to about 400. In certain embodiments, the weight ratio of the ascorbic acid to the quinone compound as used in a method provided herein is ranging from about 1 to about 1,000. In certain embodiments, the weight ratio of the ascorbic acid to the quinone compound as used in a method provided herein is ranging from about 10 to about 500. In certain embodiments, the weight ratio of the ascorbic acid to the quinone compound as used in a method provided herein is ranging from about 50 to about 500. In certain embodiments, the weight ratio of the ascorbic acid to the quinone compound as used in a method provided herein is ranging from about 100 to about 500. In certain embodiments, the weight ratio of the ascorbic acid to the quinone compound as used in a method provided herein is ranging from about 200 to about 500. In certain embodiments, the weight ratio of the ascorbic acid to the quinone compound as used in a method provided herein is ranging from about 200 to about 400. In certain embodiments, the weight ratio of the ascorbic acid to the quinone compound as used in a method provided herein is about 100, about 200, about 300, about 400, about 500, or about 600. In certain embodiments, the weight ratio of the ascorbic acid to the quinone compound as used in a method provided herein is about 200. In certain embodiments, the weight ratio of the ascorbic acid to the quinone compound as used in a method provided herein is about 400.

In certain embodiments, the weight ratio of the ascorbic acid to the quinone compound as used in a method provided herein is ranging from about 25 to about 250, from about 50 to about 200, from about 50 to about 150, or from about 80 to about 120. In certain embodiments, the weight ratio of the ascorbic acid to the quinone compound as used in a method provided herein is ranging from about 25 to about 250. In certain embodiments, the weight ratio of the ascorbic acid to the quinone compound as used in a method provided herein is ranging from about 50 to about 200. In certain embodiments, the weight ratio of the ascorbic acid to the quinone compound as used in a method provided herein is ranging from about 50 to about 150. In certain embodiments, the weight ratio of the ascorbic acid to the quinone compound as used in a method provided herein is ranging from about 80 to about 120. In certain embodiments, the weight ratio of the ascorbic acid to the quinone compound as used in a method provided herein is about 1, about 2, about 4, about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, or about 250. In certain embodiments, the weight ratio of the ascorbic acid to the quinone compound as used in a method provided herein is about 50. In certain embodiments, the weight ratio of the ascorbic acid to the quinone compound as used in a method provided herein is about 100.

In certain embodiments, the ascorbic acid as used in a method provided herein is administered to the subject in an amount ranging from about 0.1 to about 500 mg/kg/day, from about 0.2 to about 200 mg/kg/day, from about 0.5 to about 100 mg/kg/day, or from about 1 to about 100 mg/kg/day. In certain embodiments, the ascorbic acid as used in a method provided herein is administered to the subject in an amount ranging from about 0.1 to about 500 mg/kg/day. In certain embodiments, the ascorbic acid as used in a method provided herein is administered to the subject in an amount ranging from about 0.2 to about 200 mg/kg/day. In certain embodiments, the ascorbic acid as used in a method provided herein is administered to the subject in an amount ranging from about 0.5 to about 100 mg/kg/day. In certain embodiments, the ascorbic acid as used in a method provided herein is administered to the subject in an amount ranging from about 1 to about 100 mg/kg/day. In certain embodiments, the ascorbic acid as used in a method provided herein is administered to the subject in an amount of about 0.5 mg/kg/day, about 1 mg/kg/day, about 2 mg/kg/day, about 5 mg/kg/day, about 10 mg/kg/day, or about 20 mg/kg/day.

In certain embodiments, the ascorbic acid as used in a method provided herein is administered to the subject in an amount ranging from about 0.1 to about 500 mg/kg per treatment, from about 0.2 to about 200 mg/kg per treatment, from about 0.5 to about 100 mg/kg per treatment, or from about 1 to about 100 mg/kg per treatment. In certain embodiments, the ascorbic acid as used in a method provided herein is administered to the subject in an amount ranging from about 0.1 to about 500 mg/kg per treatment. In certain embodiments, the ascorbic acid as used in a method provided herein is administered to the subject in an amount ranging from about 0.2 to about 200 mg/kg per treatment. In certain embodiments, the ascorbic acid as used in a method provided herein is administered to the subject in an amount ranging from about 0.5 to about 100 mg/kg per treatment. In certain embodiments, the ascorbic acid as used in a method provided herein is administered to the subject in an amount ranging from about 1 to about 100 mg/kg per treatment. In certain embodiments, the ascorbic acid as used in a method provided herein is administered to the subject in an amount of about 0.5 mg/kg per treatment, about 1 mg/kg per treatment, about 2 mg/kg per treatment, about 5 mg/kg per treatment, about 10 mg/kg per treatment, or about 20 mg/kg per treatment.

In certain embodiments, the quinone compound as used in a method provided herein is administered to the subject in an amount ranging from about 0.001 to about 5 mg/kg/day, from about 0.002 to about 2 mg/kg/day, from about 0.005 to about 1 mg/kg/day, or from about 0.01 to about 1 mg/kg/day. In certain embodiments, the quinone compound as used in a method provided herein is administered to the subject in an amount ranging from about 0.001 to about 5 mg/kg/day. In certain embodiments, the quinone compound as used in a method provided herein is administered to the subject in an amount ranging from about 0.002 to about 2 mg/kg/day. In certain embodiments, the quinone compound as used in a method provided herein is administered to the subject in an amount ranging from about 0.005 to about 1 mg/kg/day. In certain embodiments, the quinone compound as used in a method provided herein is administered to the subject in an amount ranging from about 0.01 to about 1 mg/kg/day. In certain embodiments, the quinone compound as used in a method provided herein is administered to the subject in an amount of about 0.01 mg/kg/day, about 0.02 mg/kg/day, about 0.05 mg/kg/day, or about 0.1 mg/kg/day.

In certain embodiments, the quinone compound as used in a method provided herein is administered to the subject in an amount ranging from about 0.001 to about 5 mg/kg per treatment, from about 0.002 to about 2 mg/kg per treatment, from about 0.005 to about 1 mg/kg per treatment, or from about 0.01 to about 1 mg/kg per treatment. In certain embodiments, the quinone compound as used in a method provided herein is administered to the subject in an amount ranging from about 0.001 to about 5 mg/kg per treatment. In certain embodiments, the quinone compound as used in a method provided herein is administered to the subject in an amount ranging from about 0.002 to about 2 mg/kg per treatment. In certain embodiments, the quinone compound as used in a method provided herein is administered to the subject in an amount ranging from about 0.005 to about 1 mg/kg per treatment. In certain embodiments, the quinone compound as used in a method provided herein is administered to the subject in an amount ranging from about 0.01 to about 1 mg/kg per treatment. In certain embodiments, the quinone compound as used in a method provided herein is administered to the subject in an amount of about 0.01 mg/kg per treatment, about 0.02 mg/kg per treatment, about 0.05 mg/kg per treatment, or about 0.1 mg/kg per treatment.

The administered doses of the ascorbic acid and quinone compound can also each independently be expressed in units other than the unit "mg/kg/day" or "g/kg/day." For example, doses for parenteral administration can be expressed as mg/m²/day. One of ordinary skill in the art would readily know how to convert doses from mg/kg/day to mg/m²/day, given either the height or weight of a subject or both. For example, a dose of 1 mg/kg/day for a 65 kg human is approximately equal to 38 mg/m²/day.

The ascorbic acid used in the method provided herein is administered intratumorally. In certain embodiments, the ascorbic acid used in a method provided herein is administered by intratumoral infusion. In certain embodiments, the ascorbic acid used in a method provided herein is administered intratumorally *via* direct injection. In certain embodiments, the ascorbic acid used in a method provided herein is administered intratumorally *via* intracavitary delivery. In certain embodiments, the ascorbic acid used in a method provided herein is administered intratumorally *via* intracerebral delivery. In certain embodiments, the ascorbic acid used in a method provided herein is administered intratumorally *via* intracranial delivery. In certain embodiments, the ascorbic acid used in a method provided herein is administered intratumorally *via* intrathecal delivery. In certain embodiments, the ascorbic acid used in a method provided herein is administered intratumorally *via* intraventricular delivery. In certain embodiments, the ascorbic acid used in a method provided herein is administered intratumorally *via* convection-enhanced delivery (CED).

The quinone compound used in the method provided herein is administered intratumorally. In certain embodiments, the quinone compound used in a method provided herein is administered by intratumoral infusion. In certain embodiments, the quinone compound used in a method provided herein is administered intratumorally *via* direct injection. In certain embodiments, the quinone compound used in a method provided herein is administered intratumorally *via* intracavitary delivery. In certain embodiments, the quinone compound used in a method provided herein is administered intratumorally *via* intracerebral delivery. In certain embodiments, the quinone compound used in a method provided herein is administered intratumorally *via* intracranial delivery. In certain embodiments, the quinone compound used in a method provided herein is administered intratumorally *via* intrathecal delivery. In certain embodiments, the quinone compound used in a method provided herein is administered intratumorally *via* intraventricular delivery. In certain embodiments, the quinone compound used in a method provided herein is administered intratumorally *via* CED.

The ascorbic acid and quinone compound used in the method provided herein are administered together intratumorally. In certain embodiments, the ascorbic acid and quinone compound used in a method provided herein are administered by intratumoral infusion. In certain embodiments, the ascorbic acid and quinone compound used in a method provided herein are administered together intratumorally *via* direct injection. In certain embodiments, the ascorbic acid and quinone compound used in a method provided herein are administered together intratumorally *via* implantation. In certain embodiments, the ascorbic acid and quinone compound used in a method provided herein are administered together intratumorally *via* intracavitary delivery. In certain embodiments, the ascorbic acid and quinone compound used in a method provided herein are administered together intratumorally *via* intracerebral delivery. In certain embodiments, the ascorbic acid and quinone compound used in a method provided herein are administered together intratumorally *via* intracranial delivery. In certain embodiments, the ascorbic acid and quinone compound used in a method provided herein are administered together intratumorally *via* intrathecal delivery. In certain embodiments, the ascorbic acid and quinone compound used in a method provided herein are administered together intratumorally *via* intraventricular delivery. In certain embodiments, the ascorbic acid and quinone compound used in a method provided herein are administered together intratumorally *via* CED.

In certain embodiments, the ascorbic acid and quinone compound as used in a method provided herein are administered once a month, once every two weeks, once a week, or once daily (QD). In addition, the administration can be continuous, *i.e.,* every day, or intermittently. The term "intermittent" or "intermittently" as used herein is intended to mean stopping and starting at either regular or irregular intervals. For example, intermittent administration of a compound provided herein is administration for one to six days per week, administration in cycles (*e.g.*, daily administration for two to eight consecutive weeks, then a rest period with no administration for up to one week), or administration on alternate days.

In certain embodiments, the ascorbic acid and quinone compound as used in a method provided herein are cyclically administered to a subject. Cycling therapy involves the administration of active agent(s) for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

In certain embodiments, the ascorbic acid and quinone compound are administered repetitively if necessary, for example, until the subject experiences stable disease or regression, or until the subject experiences disease progression or unacceptable toxicity. Stable disease or lack thereof is determined by a method known in the art such as evaluation of subject's symptoms, physical examination, or diagnostic testing.

The ascorbic acid and quinone compound as used in a method provided herein can also be combined or used in combination with a second therapeutic agent useful in the treatment of the cancer described herein. In certain embodiments, the second therapeutic agent is an anticancer agent. In certain embodiments, the second therapeutic agent is bevacizumab, carmustine, everolimus, lomustine, or temozolomide. In certain embodiments, the second therapeutic agent is a farnesyltransferase inhibitor. In certain embodiments, the farnesyltransferase inhibitor is gingerol, gliotoxin, lonafarnib, tipifarnib, FTI-277, GGTI-287, GGTI-2418, or L-778123. In certain embodiments, the farnesyltransferase inhibitor is lonafarnib or tipifarnib.

In certain embodiments, the methods provided herein encompass treating a subject regardless of patient's age. In certain embodiments, the subject is a pediatric patient. In certain embodiments, the subject is an adult patient.

In certain embodiments, the subject is a mammal. In certain embodiments, the subject is a human.

In certain embodiments, provided herein are kits which, when used by the medical practitioner, can simplify the administration of appropriate amounts of active ingredients to a subject. In certain embodiments, the kit provided herein includes containers and dosage forms of the active ingredients provided herein. Kits provided herein can further include devices that are used to administer the active ingredients.

The disclosure will be further understood by the following non-limiting examples.

### EXAMPLES

As used herein, the symbols and conventions used in the examples, regardless of whether a particular abbreviation is specifically defined, are consistent with those used in the contemporary scientific literature, for example, the Journal of the American Chemical Society, the Journal of Medicinal Chemistry, or the Journal of Biological Chemistry. Specifically, but without limitation, the following abbreviations may be used in the examples and throughout the specification: kg (kilograms); g (grams); mg (milligrams); µg (micrograms); L (liters); mL (milliliters); µL (microliters); mM (millimolar); µM (micromolar); mmol (millimoles); h (hour or hours); and MRI (magnetic resonance imaging).

### Example 1

### Antiproliferative activity of vitamin C and/or compound A6 against cancerous cells

The antiproliferative activity of vitamin C (L-ascorbic acid) and/or compound **A6** was determined in Colon26 (colon cancer), GS9L (glioblastoma), Jurkat (leukemia), MCF7 (breast cancer), and U87MG (glioblastoma). Jurkat cells were cultured in RPMI-1640 medium supplemented with 10% heat-inactivated fetal bovine serum (FBS), penicillin (100 U/mL), and streptomycin (100 µg/mL). Normal lymphocytes were cultured in the same medium supplemented with 10% FBS only. Adhesive cancer cell lines (Colon26, GS9L, MCF7, and U87MG) were cultured in DMEM supplemented with 10% FBS, penicillin (100 U/mL), and streptomycin (100 µg/mL). Adhesive non-cancer cell lines, FHC and MCF10A, were cultured in DMEM-F12 and DMEM, respectively, each supplemented with 10% FBS and growth factors. EOC2 cells were cultured in DMEM supplemented with 10% FBS. All cells were cultured in a humidified atmosphere containing 5% CO₂ at 37 °C.

The adhesive cells were released using a trypsin-EDTA solution (0.05% of trypsin/EDTA) and subsequently washed with PBS. The cells were collected by centrifugation (1,000 × g/10 min for leukemia lymphocytes; 1,500 × g/15 min for normal lymphocytes; and 800 × g/5 min for adhesive cells) and placed in a fresh medium without antibiotics and growth factors prior to treatment with vitamin C and/or compound **A6**. The cells (1 × 10⁶ cells/mL for non-adhesive and 3 × 10⁵ cells/mL for adhesive) were incubated with vitamin C and/or compound **A6** for different time intervals in a cell incubator. At each time interval, aliquots were removed for analyses.

For lymphocytes, cell proliferation and viability were analyzed by trypan blue staining and automated counting using a COUNTESS automated cell counter. Briefly, 10 µL of trypan blue (0.4%) was added to 10 µL of a cell suspension and incubated for 30 s, and 10 µL of the cell suspension was placed in a COUNTESS glass chamber. The numbers of live and dead cells in the suspension were counted automatically. The linear range of operation with the automated cell counter was 1 × 10⁴-5 × 10⁶ cells/mL, and the optimal cell size was in the range of 5-60 µm.

For other cell lines, cell proliferation and viability were analyzed using the CELLTITER-GLO luminescent cell viability assay. Briefly, 100 µL aliquots of a cell suspension were placed in a well of a 96-well plate and incubated with vitamin C and/or compound A6 for 24 and 48 h in a humidified atmosphere containing 5% CO₂ at 37 °C. The CELLTITER-GLO reagent containing luciferin and luciferase (100 µL) was added to each well, followed by incubation. The luminescence produced by the luciferase-catalyzed conversion of luciferin into oxyluciferin by living cells was detected using a microplate reader in a chemiluminescent mode. The results are summarized in Tables 1 and 2.

**TABLE 1. Antiproliferative activity of a combination of vitamin C and compound A6 (weight ratio: 100: 1)**

| **Cell Line** | **IC₅₀ (µM)** | |
|---|---|---|
| | **Vitamin C** | **Compound A6** |
| Normal lymphocytes | > 20 | > 2,000 |
| Leukemia T lymphocytes (Jurkat) | 3 | 300 |
| Lymphoma B lymphocytes (Raji) | 5 | 500 |
| Normal colon epithelial cells (FHC) | > 20 | > 2,000 |
| Colon cancer cells (Colon 26) | 4.8 | 480 |
| Normal breast epithelial cells (MCF10A) | > 20 | > 2,000 |
| Breast cancer cells (MCF7) | 3.5 | 350 |
| Normal microglial cells | > 20 | > 2,000 |
| Human glioblastoma cells (U87MG) | 11 | 1,100 |

**TABLE 2. Antiproliferative activity of vitamin C and compound A6, alone or in combination**

| **Compound(s)** | **IC₅₀ (µM)** | | |
|---|---|---|---|
| | **Jurkat** | **Raji** | **Colon26** |
| Vitamin C | 2,000 | > 2,000 | > 2,000 |
| Compound **A6** | 5 | > 20 | > 20 |
| Vitamin C/Compound **A6** | 200/2 | 500/5 | 480/4.8 |

### Example 2

### Antitumor activity of vitamin C and compound A6 in a xenograft mouse model

Six-week-old male Balb/c nude mice were maintained in specific pathogen-free conditions. Glioblastoma cells (U-87 MG) were cultured in DMEM supplemented with 10% FBS, penicillin (100 U/mL), and streptomycin (100 mg/L) at 37 °C in a humidified atmosphere containing 5% CO₂. After deep anesthesia, the mice were positioned in a stereotactic frame. A small craniectomy was performed at 2-3 mm from the midline and 1 mm anterior to the coronal suture. The glioblastoma cells (5 × 10⁵ cells in 5 µL) were stereotactically injected into the brain parenchyma at a depth of 3 mm on Day 1 using an automated injector.

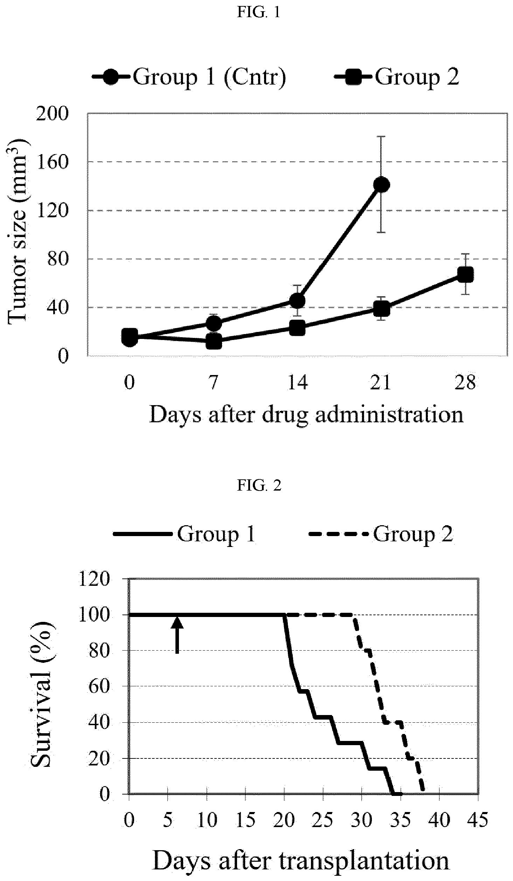

Vitamin C (L-ascorbic acid) and compound **A6** were dissolved in PBS at 10 mM (pH 7.4). On Day 7, the mice were treated *via* intracranial injection with (i) a saline solution (5 µL) in Group 1 of 7 mice as a control; or (ii) a solution (5 µL) containing L-ascorbic acid (7 mg/kg) and compound **A6** (70 µg/kg) in Group 2 of 5 mice. After the intracranial treatment, the glioblastoma-bearing mice in the control group were fed with regular water and the glioblastoma-bearing mice in the treatment group were fed orally with water containing L-ascorbic acid (15 g/L) and menadione (150 mg/L) daily. The glioblastoma-bearing mice were analyzed on Days 7, 14, 21, 28, and 35 (*i.e.,* 0, 7, 14, 21, and 28 days after drug administration) by magnetic resonance imaging (MRI) to determine the size of the glioblastoma. The survival of the glioblastoma-bearing mice was also followed. The results are summarized in FIGS. 1 and 2.

### Example 3

### Antitumor activity of vitamin C and compound A6 in a xenograft mouse model

Six-week-old male Balb/c nude mice were maintained in specific pathogen-free conditions. Glioblastoma cells (U-87 MG) were cultured in DMEM supplemented with 10% FBS, penicillin (100 U/mL), and streptomycin (100 mg/L) at 37 °C in a humidified atmosphere containing 5% CO₂. The glioblastoma cells (5 × 10⁵ cells in 5 µL) were injected subcutaneously into a leg of each anesthetized mouse on Day 1.

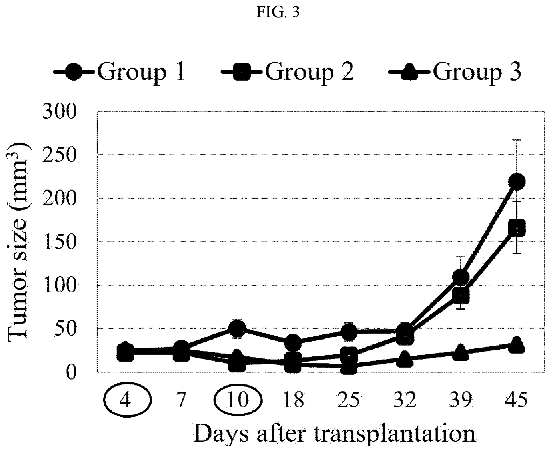

Vitamin C (L-ascorbic acid) and compound **A6** were dissolved in PBS at 10 mM (pH 7.4). The mice were divided into three groups with two mice in each group. The mice were treated subcutaneously with (i) a saline solution (50 µL) in Group 1 as a control *via* intratumoral injection on Day 4; (ii) a solution (50 µL) containing L-ascorbic acid (7 mg/kg) and compound **A6** (70 µg/kg) in Group 2 *via* intratumoral injection on Day 4; and (iii) a solution (50 µL) containing L-ascorbic acid (7 mg/kg) and compound **A6** (70 µg/kg) per treatment in Group 3 *via* intratumoral injection on Day 4 and near the tumor on Day 11. Tumor sizes (length (L) and width (W)) are measured using a digital caliper, and the tumor volume were calculated (L × W × W)/2. The results are summarized in FIG. 3.

The examples set forth above are provided to give those of ordinary skill in the art with a complete disclosure and description of how to make and use the claimed embodiments, and are not intended to limit the scope of what is disclosed herein.

## Claims

1. A combination of
(i) ascorbic acid, or a diastereomer, a mixture of two or more diastereomers, a tautomer, a mixture of two or more tautomers; or a pharmaceutically acceptable salt, solvate, or hydrate thereof; and
(ii) a quinone compound, or a tautomer, a mixture of two or more tautomers, or an isotopic variant thereof; or a pharmaceutically acceptable salt, solvate or hydrate thereof; wherein the quinone compound is 2,3-dimethoxy-5-methyl-1,4-benzoquinone; for use in a method of
treating or alleviating one or more symptoms of cancer in a subject, comprising administering intratumorally to the subject in need thereof a therapeutically effective amount of (i) and (ii).

2. The combination for use according to claim 1, wherein
the cancer is a solid cancer.

3. The combination for use according to claim 1 or 2, wherein the cancer is bladder cancer, brain cancer, breast cancer, colon cancer, endometrial cancer, kidney cancer, liver cancer, lung cancer, lymphoma, melanoma, non-Hodgkin lymphoma, ovarian cancer, pancreatic cancer, prostate cancer, rectal cancer, stomach cancer, or thyroid cancer.

4. The combination for use according to any one of claims 1 to 3, wherein the cancer is brain cancer; or wherein the cancer is a primary or secondary brain cancer; or wherein the cancer is anaplastic astrocytoma, anaplastic ependymoma, anaplastic oligodendroglioma, craniopharyngioma, diffuse astrocytoma, ependymoblastoma, gangliocytoma, ganglioglioma, glioblastoma, medulloblastoma, pineoblastoma, pineocytoma, pilocytic astrocytoma, or pure oligodendroglioma.

5. The combination for use according to any one of claims 1 to 4, wherein the cancer is a glioma; or wherein the cancer is astrocytoma, glioblastoma, meningioma, or medulloblastoma.

6. The combination for use according to any one of claims 1 to 5, wherein the cancer is glioblastoma.

7. The combination for use according to any one of claims 1 to 6, wherein
(1) the cancer is resectable or unresectable; and/or
(2) the cancer is recurrent; and/or
(3) the cancer is relapsed or refractory; and/or
(4) the cancer is metastatic; and/or
(5) the cancer is drug-resistant.

8. The combination for use according to any one of claims 1 or 7, wherein the ascorbic acid is L-ascorbic acid or a pharmaceutically acceptable salt thereof, or a pharmaceutically acceptable solvate or hydrate thereof, wherein the pharmaceutically acceptable salt of ascorbic acid optionally is sodium L-ascorbate, potassium L-ascorbate, calcium L-ascorbate, or magnesium L-ascorbate, or a pharmaceutically acceptable solvate or hydrate thereof; or a mixture thereof, optionally
(i) sodium L-ascorbate, or a pharmaceutically acceptable solvate or hydrate thereof;
(ii) potassium L-ascorbate, or a pharmaceutically acceptable solvate or hydrate thereof;
(iii) calcium L-ascorbate, or a pharmaceutically acceptable solvate or hydrate thereof; or
(iv) magnesium L-ascorbate, or a pharmaceutically acceptable solvate or hydrate thereof.

9. The combination for use according to any one of claims 1 to 8, wherein the weight ratio of the ascorbic acid to the quinone compound is ranging from about 10 to about 500, or is about 50, about 100, about 200, or about 400.

10. The combination for use according to any one of claims 1 to 9, wherein
(1) the ascorbic acid is administered *via* direction injection;
(2) the ascorbic acid is administered *via* intracerebral delivery;
(3) the ascorbic acid is administered *via* intracranial delivery;
(4) the ascorbic acid is administered *via* convection-enhanced delivery; or
(5) the ascorbic acid is administered *via* implantation.

11. The combination for use according to any one of claims 1 to 10, wherein
(1) the quinone compound is administered *via* direction injection;
(2) the quinone compound is administered *via* intracerebral delivery;
(3) the quinone compound is administered *via* intracranial delivery;
(4) the quinone compound is administered *via* convection-enhanced delivery; or
(5) the quinone compound is administered *via* implantation.

12. The combination for use according to any one of claims 1 to 11, wherein
(1) the ascorbic acid and quinone compound are administered via direction injection;
(2) the ascorbic acid and quinone compound are administered via intracerebral delivery;
(3) the ascorbic acid and quinone compound are administered via intracranial delivery;
(4) the ascorbic acid and quinone compound are administered via convection-enhanced delivery; or
(5) the ascorbic acid and quinone compound are administered via implantation.

13. The combination for use according to any one of claims 1 to 12, wherein the ascorbic acid and quinone compound are administered together.

14. The combination for use according to any one of claims 1 to 13, wherein the combination further comprises a second therapeutic agent and the method further comprises administering to the subject in need thereof a therapeutically effective amount of said second therapeutic agent.

15. The combination for use according to claim 14, wherein
(1) the second therapeutic agent is an anticancer agent; and/or
(2) the second therapeutic agent is bevacizumab, carmustine, everolimus, lomustine, or temozolomide; and/or
(3) the second therapeutic agent is a farnesyltransferase inhibitor, optionally lonafamib or tipifamib.

## Patentansprüche

1. Eine Kombination aus
(i) Ascorbinsäure oder einem Diastereomer, einer Mischung aus zwei oder mehr Diastereomeren, einem Tautomer, einer Mischung aus zwei oder mehr Tautomeren oder einem pharmazeutisch annehmbaren Salz, Solvat oder Hydrat davon; und
(ii) einer Chinonverbindung oder einem Tautomer, einer Mischung aus zwei oder mehr Tautomeren oder einer Isotopenvariante davon oder einem pharmazeutisch annehmbaren Salz, Solvat, oder Hydrat davon, wobei die Chinonverbindung 2,3-Dimethoxy-5-methyl-1,4-benzochinon ist;
zur Verwendung in einem Verfahren zur Behandlung oder Linderung eines oder mehrerer Krebssymptome in einem Patienten, umfassend die intratumorale Verabreichung einer therapeutisch wirksamen Menge von (i) und (ii) an den Patienten, welcher dieser bedarf.

2. Die Kombination zur Verwendung gemäß Anspruch 1, wobei der Krebs ein solider Krebs ist.

3. Die Kombination zur Verwendung gemäß Anspruch 1 oder 2, wobei der Krebs Blasenkrebs, Hirnkrebs, Brustkrebs, Dickdarmkrebs, Endometriumkrebs, Nierenkrebs, Leberkrebs, Lungenkrebs, Lymphom, Melanom, Non-Hodgkin-Lymphom, Eierstockkrebs, Bauchspeicheldrüsenkrebs, Prostatakrebs, Rektalkrebs, Magenkrebs oder Schilddrüsenkrebs ist.

4. Die Kombination zur Verwendung gemäß einem der Ansprüche 1 bis 3, wobei der Krebs Hirnkrebs ist; oder wobei der Krebs ein primärer oder sekundärer Hirnkrebs ist; oder wobei der Krebs ein anaplastisches Astrozytom, ein anaplastisches Ependymom, ein anaplastisches Oligodendrogliom, ein Kraniopharyngiom, ein diffuses Astrozytom, ein Ependymoblastom, ein Gangliozytom, ein Gangliogliom, ein Glioblastom, ein Medulloblastom, ein Pineoblastom, ein Pineozytom, ein pilozytisches Astrozytom oder ein reines Oligodendrogliom ist.

5. Die Kombination zur Verwendung gemäß einem der Ansprüche 1 bis 4, wobei der Krebs ein Gliom ist; oder wobei der Krebs ein Astrozytom, Glioblastom, Meningiom oder Medulloblastom ist.

6. Die Kombination zur Verwendung gemäß einem der Ansprüche 1 bis 5, wobei der Krebs ein Glioblastom ist.

7. Die Kombination zur Verwendung gemäß einem der Ansprüche 1 bis 6, wobei
(1) der Krebs resektabel oder nicht resektabel ist; und/oder
(2) der Krebs rezidivierend ist; und/oder
(3) der Krebs rezidiviert oder refraktär ist; und/oder
(4) der Krebs metastasiert ist; und/oder
(5) der Krebs arzneimittelresistent ist.

8. Die Kombination zur Verwendung gemäß einem der Ansprüche 1 oder 7, wobei die Ascorbinsäure L-Ascorbinsäure oder ein pharmazeutisch annehmbares Salz davon oder ein pharmazeutisch annehmbares Solvat oder Hydrat davon ist, wobei das pharmazeutisch annehmbare Salz der Ascorbinsäure gegebenenfalls Natrium-L-Ascorbat, Kalium-L-Ascorbat, Calcium-L-Ascorbat oder Magnesium-L-Ascorbat oder ein pharmazeutisch annehmbares Solvat oder Hydrat davon ist; oder eine Mischung davon, gegebenenfalls
(i) Natrium-L-Ascorbat oder ein pharmazeutisch akzeptables Solvat oder Hydrat davon;
(ii) Kalium-L-Ascorbat oder ein pharmazeutisch akzeptables Solvat oder Hydrat davon;
(iii) Calcium-L-Ascorbat oder ein pharmazeutisch akzeptables Solvat oder Hydrat davon; oder
(iv) Magnesium-L-Ascorbat, oder ein pharmazeutisch akzeptables Solvat oder Hydrat davon.

9. Die Kombination zur Verwendung gemäß einem der Ansprüche 1 bis 8, wobei das Gewichtsverhältnis der Ascorbinsäure zu der Chinonverbindung im Bereich von ungefähr 10 bis ungefähr 500 liegt, oder ungefähr 50, ungefähr 100, ungefähr 200 oder ungefähr 400 beträgt.

10. Die Kombination zur Verwendung gemäß einem der Ansprüche 1 bis 9, wobei
(1) die Ascorbinsäure durch direkte Injektion verabreicht wird;
(2) die Ascorbinsäure durch intrazerebrale Zuführung verabreicht wird;
(3) die Ascorbinsäure durch intrakranielle Zuführung verabreicht wird;
(4) die Ascorbinsäure durch konvektionsverstärkte Zuführung verabreicht wird; oder
(5) die Ascorbinsäure durch Implantation verabreicht wird.

11. Die Kombination zur Verwendung gemäß einem der Ansprüche 1 bis 10, wobei
(1) die Chinonverbindung durch direkte Injektion verabreicht wird;
(2) die Chinonverbindung durch intrazerebrale Zuführung verabreicht wird;
(3) die Chinonverbindung durch intrakranielle Zuführung verabreicht wird;
(4) die Chinonverbindung durch konvektionsverstärkte Zuführung verabreicht wird; oder
(5) die Chinonverbindung durch Implantation verabreicht wird.

12. Die Kombination zur Verwendung gemäß einem der Ansprüche 1 bis 11, wobei
(1) die Ascorbinsäure und die Chinonverbindung durch direkte Injektion verabreicht werden;
(2) die Ascorbinsäure und die Chinonverbindung durch intrazerebrale Zuführung verabreicht werden;
(3) die Ascorbinsäure und die Chinonverbindung durch intrakranielle Zuführung verabreicht werden;
(4) die Ascorbinsäure und die Chinonverbindung durch konvektionsverstärkte Zuführung verabreicht werden; oder
(5) die Ascorbinsäure und die Chinonverbindung durch Implantation verabreicht werden.

13. Die Kombination zur Verwendung gemäß einem der Ansprüche 1 bis 12, wobei die Ascorbinsäure und die Chinonverbindung zusammen verabreicht werden.

14. Die Kombination zur Verwendung gemäß einem der Ansprüche 1 bis 13, wobei die Kombination des Weiteren einen zweiten therapeutischen Wirkstoff umfasst und das Verfahren des Weiteren die Verabreichung einer therapeutisch wirksamen Menge des zweiten therapeutischen Wirkstoffs an den Patienten, welcher dieser bedarf, umfasst.

15. Die Kombination zur Verwendung gemäß Anspruch 14, wobei
(1) der zweite therapeutische Wirkstoff ein Antikrebswirkstoff ist; und/oder
(2) der zweite therapeutische Wirkstoff Bevacizumab, Carmustin, Everolimus, Lomustin oder Temozolomid ist; und/oder
(3) der zweite therapeutische Wirkstoff ein Farnesyltransferase-Inhibitor, gegebenenfalls Lonafarnib oder Tipifarnib ist.

## Revendications

1. Combinaison de
(i) acide ascorbique, ou d'un diastéréomère, d'un mélange de deux ou plus de deux diastéréomères, d'un tautomère, d'un mélange de deux ou plus de deux tautomères ; ou d'un sel, solvate ou hydrate pharmaceutiquement acceptable de ceux-ci ; et
(ii) d'un composé de quinone, ou d'un tautomère, d'un mélange de deux ou plus de deux tautomères, ou d'un variant isotopique de ceux-ci ; ou d'un sel, solvate ou hydrate pharmaceutiquement acceptable de ceux-ci ; sachant que le composé de quinone est du 2,3-diméthoxy-5-méthyl-1,4-benzoquinone ;
pour utilisation dans un procédé de traitement ou de soulagement d'un ou de plusieurs symptômes de cancer chez un sujet, comprenant l'administration par voie intratumorale au sujet qui en a besoin d'une quantité thérapeutiquement efficace de (i) et (ii).

2. La combinaison pour utilisation selon la revendication 1, sachant que le cancer est un cancer solide.

3. La combinaison pour utilisation selon la revendication 1 ou 2, sachant que le cancer est un cancer de la vessie, un cancer du cerveau, un cancer du sein, un cancer du colon, un cancer de l'endomètre, un cancer du rein, un cancer du foie, un cancer du poumon, un lymphome, un mélanome, un lymphome non hodgkinien, un cancer de l'ovaire, un cancer du pancréas, un cancer de la prostate, un cancer du rectum, un cancer de l'estomac, ou un cancer de la thyroïde.

4. La combinaison pour utilisation selon l'une quelconque des revendications 1 à 3, sachant que le cancer est un cancer du cerveau ; ou sachant que le cancer est un cancer du cerveau primaire ou secondaire ; ou sachant que le cancer est un astrocytome anaplasique, un épendymome anaplasique, un oligodendrogliome anaplasique, un craniopharyngiome, un astrocytome diffus, un épendymoblastome, un glangliocytome, un gangliogliome, un glioblastome, un médulloblastome, un pinéoblastome, un pinéocytome, un astrocytome pilocytique, ou un oligodendrogliome pur.

5. La combinaison pour utilisation selon l'une quelconque des revendications 1 à 4, sachant que le cancer est un gliome ; ou sachant que le cancer est un astrocytome, un glioblastome, un méningiome, ou un médulloblastome.

6. La combinaison pour utilisation selon l'une quelconque des revendications 1 à 5, sachant que le cancer est un glioblastome.

7. La combinaison pour utilisation selon l'une quelconque des revendications 1 à 6, sachant que
(1) le cancer est résécable ou non résécable ; et/ou
(2) le cancer est récurrent ; et/ou
(3) le cancer est récidivant ou réfractaire ; et/ou
(4) le cancer est métastatique ; et/ou
(5) le cancer est résistant aux médicaments.

8. La combinaison pour utilisation selon l'une quelconque des revendications 1 ou 7, sachant que l'acide ascorbique est de l'acide L-ascorbique ou un sel pharmaceutiquement acceptable de celui-ci, ou un solvate ou hydrate pharmaceutiquement acceptable de celui-ci, sachant que le sel pharmaceutiquement acceptable d'acide ascorbique est facultativement du L-ascorbate de sodium, du L-ascorbate de potassium, du L-ascorbate de calcium, ou du L-ascorbate de magnésium, ou un solvate ou hydrate pharmaceutiquement acceptable de ceux-ci ; ou un mélange de ceux-ci, facultativement
(i) du L-ascorbate de sodium, ou un solvate ou hydrate pharmaceutiquement acceptable de celui-ci ;
(ii) du L-ascorbate de potassium, ou un solvate ou hydrate pharmaceutiquement acceptable de celui-ci ;
(iii) du L-ascorbate de calcium, ou un solvate ou hydrate pharmaceutiquement acceptable de celui-ci ; ou
(iv) du L-ascorbate de magnésium, ou un solvate ou hydrate pharmaceutiquement acceptable de celui-ci.

9. La combinaison pour utilisation selon l'une quelconque des revendications 1 à 8, sachant que le rapport de poids de l'acide ascorbique au composé de quinone va d'environ 10 à environ 500, ou est d'environ 50, d'environ 100, d'environ 200, ou d'environ 400.

10. La combinaison pour utilisation selon l'une quelconque des revendications 1 à 9, sachant que
(1) l'acide ascorbique est administré par injection directionnelle ;
(2) l'acide ascorbique est administré par délivrance intracérébrale ;
(3) l'acide ascorbique est administré par délivrance intracrânienne ;
(4) l'acide ascorbique est administré par délivrance améliorée par convection ; ou
(5) l'acide ascorbique est administré par implantation.

11. La combinaison pour utilisation selon l'une quelconque des revendications 1 à 10, sachant que
(1) le composé de quinone est administré par injection directionnelle ;
(2) le composé de quinone est administré par délivrance intracérébrale ;
(3) le composé de quinone est administré par délivrance intracrânienne ;
(4) le composé de quinone est administré par délivrance améliorée par convection ; ou
(5) le composé de quinone est administré par implantation.

12. La combinaison pour utilisation selon l'une quelconque des revendications 1 à 11, sachant que
(1) l'acide ascorbique et le composé de quinone sont administrés par injection directionnelle ;
(2) l'acide ascorbique et le composé de quinone sont administrés par délivrance intracérébrale ;
(3) l'acide ascorbique et le composé de quinone sont administrés par délivrance intracrânienne ;
(4) l'acide ascorbique et le composé de quinone sont administrés par délivrance améliorée par convection ; ou
(5) l'acide ascorbique et le composé de quinone sont administrés par implantation.

13. La combinaison pour utilisation selon l'une quelconque des revendications 1 à 12, sachant que l'acide ascorbique et le composé de quinone sont administrés ensemble.

14. La combinaison pour utilisation selon l'une quelconque des revendications 1 à 13, sachant que la combinaison comprend en outre un deuxième agent thérapeutique et le procédé comprend en outre l'administration au sujet qui en a besoin d'une quantité thérapeutiquement efficace dudit deuxième agent thérapeutique.

15. La combinaison pour utilisation selon la revendication 14, sachant que
(1) le deuxième agent thérapeutique est un agent anticancer ; et/ou
(2) le deuxième agent thérapeutique est du bévacizumab, de la carmustine, de l'évérolimus, de la lomustine, ou du témozolomide ; et/ou
(3) le deuxième agent thérapeutique est un inhibiteur de farnesyltransférase, facultativement du lonafarnib ou du tipifarnib.
